# EUROPEAN PATENT SPECIFICATION

(11) **EP 3 297 562 B1**
(45) Date of publication and mention of the grant of the patent: **12.02.2025**
(21) Application number: 16723708.0
(22) Date of filing: 11.05.2016
(51) Int. Cl.: A61B 34/10, A61B 34/20, G06T 7/12, G06T 7/155, G06T 7/73, A61B 90/00, A61B 17/00

(54) **INTRA-PROCEDURAL ACCURACY FEEDBACK FOR IMAGE-GUIDED BIOPSY**
INTRAPROZEDURALE GENAUIGKEITSRÜCKMELDUNG FÜR BILDGEFÜHRTE BIOPSIE
RÉTROACTION DE PRÉCISION PENDANT UN PROCESSUS DE BIOPSIE GUIDÉE PAR IMAGE

(30) Priority: 18.05.2015 US 201562162848 P
(43) Date of publication of application: 28.03.2018
(73) Proprietor: Koninklijke Philips N.V., 5656 AG Eindhoven (NL)
(72) Inventor: YAN, Pingkun, 5656 AE Eindhoven (NL); KRUECKER, Jochen, 5656 AE Eindhoven (NL)
(74) Representative: Philips Intellectual Property & Standards
(86) International application number: PCT/EP2016/060545
(87) International publication number: WO 2016/184746

(56) References cited:
- EP-A1- 1 504 713
- EP-A2- 1 323 380
- WO-A1-2010/069360
- WO-A1-2015/023665
- WO-A1-2016/009350
- WO-A1-97/29682
- DE-A1- 102010 039 604
- US-A1- 2009 118 640
- US-A1- 2011 137 156
- US-A1- 2013 116 548

## Description

### BACKGROUND:

### Technical Field

This disclosure relates to medical imaging and more particularly to systems and methods that provide feedback during a biopsy procedure.

### Description of the Related Art

In image-guided biopsy procedures, physicians rely on real-time imaging to guide an insertion of a biopsy gun to targets, which may be visible directly in a live image, or may be transferred from a prior image and superimposed on the live image using image fusion. Most biopsy guns have a spring-loaded mechanism that, when fired, shoots forward to obtain a tissue sample from a location that is offset from a needle tip by a fixed distance (i.e., the "throw" of the needle). Operators need to estimate that distance and position the biopsy needle proximal to the intended target, offset by the "throw", and with the needle trajectory intersecting the target. When the operator considers the needle to be positioned correctly, the biopsy gun is "fired" to obtain the tissue sample. If the distance to the target or the trajectory of the biopsy needle is not estimated correctly, the target will not be sampled accurately.

In addition, by using hardware tracking technology and fusing pre-procedural diagnostic images with intra-procedural live imaging, e.g., ultrasound, pre-identified targets from diagnostic images can be mapped to the space of live imaging. An example of such system is the Philips^{®} UroNav^{™} system, which is used for image fusion guided prostate cancer biopsy. In this system, magnetic resonance (MR) images are fused with the transrectal ultrasound (TRUS) images in real time. The suspicious prostate cancer lesions are identified as biopsy targets by radiologists on MR images. Those targets are usually not visible from TRUS images. During a prostate biopsy procedure, MR images are fused with TRUS images via electromagnetic (EM) tracking. In this way, the MR targets can be mapped to the TRUS images and thus can be superimposed over TRUS images when they are in view. With such guidance, users can aim for a target in TRUS with a biopsy gun.

However, even with the targets displayed over TRUS, there is no guarantee that a user can hit the target accurately with the biopsy needle for several reasons. For example, a biopsy needle is usually spring loaded, and the core-taking part will be fired out when a button is released. The user has to insert the needle to a certain depth but stay proximal to the target, accounting for the throw of the needle when fired. This mental estimation of the insertion depth may be error prone, and inaccurate insertion depth estimation may result in the sampled location being either too deep or too shallow and thus missing the target. Another cause for missing a target is due to the biopsy guide bending and/or shifting. When this happens, the needle will deviate from a biopsy guide line displayed on a screen. Thus, when a user uses the displayed guide line to aim for the target, the actual sample will be taken from an area other than the targeted area. Yet another factor can be the motion due to either patient movement or TRUS probe movement. During needle firing, if there is such motion, the needle may be directed away from the target.

EP1323380A2 describes a method and apparatus for enabling a biopsy needle to be observed in a three-dimensional ultrasound diagnostic system using an interventional ultrasound system.

US2013/116548A1 relates to tools to improve a 3-D image aided biopsy or treatment procedure for prostate gland. The tools include i) the identification of various parts of prostate to classify as per regular classification in pathological reports, ii) Computing and displaying the insertion depth of needle with respect to a selected target point during the procedure, iii) Computing and displaying the distance from needle tip to prostate surface following a procedure and, iv) Calibration for misalignment of a 2-D imaging transducer when used under tracked motion for a procedure.

WO2015/023665A1 describes a method of guiding an interventional instrument within a patient anatomy. The method comprises processing a target location within the patient anatomy and receiving a position for a tip portion of an interventional instrument at a first location within the patient anatomy. The method also comprises determining a three-dimensional distance between the first location and the target location and displaying a symbol representing the target location and a symbol representing the tip portion of the interventional instrument.

### SUMMARY

The invention is set out in the appended set of claims.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of arrangements with reference to the following figures wherein:
FIG. 1 is a block/flow diagram showing a feedback system for instrument guidance in accordance with one arrangement;
FIG. 2 is a block/flow diagram showing a feedback guidance module in greater detail in accordance with onearrangement;
FIG. 3 is a block/flow diagram showing image detection for an instrument in accordance with onearrangement;
FIG. 4 is a diagram showing an image having a projected biopsy guideline and a needle with a core taking projection deviating from the guideline in accordance with onearrangement;
FIG. 5 is a diagram showing an image having an instrument aligned with the projected biopsy guideline and a core taking projection displayed on the guideline in accordance with onearrangement;
FIG. 6 is a diagram showing three instances where a needle is positioned at a "good' distance from a target, "too shallow" from the target and "too deep" from the target;
FIG. 7 is a diagram showing three instances where a core-taking portion of a needle is positioned at a "good' distance from a target, "too shallow" from the target and "too deep" from the target; and
FIG. 8 is a block/flow diagram showing a feedback method for instrument guidance in accordance with onearrangement.

### DETAILED DESCRIPTION OF EMBODIMENTS

In accordance with the present principles, systems and methods are provided that produce feedback to a user before and after a biopsy gun firing by determining a spatial relationship between the biopsy needle and a target. Users will be able to take corresponding actions when needed to improve the accuracy and in turn achieve higher success rates. Inan arrangement, a system in accordance with the present principles uses image processing and analysis methods to detect a needle in real-time imaging. The system can provide visual feedback superimposed on images to show a desired position of biopsy gun before firing. This can also be used together with hardware-based device tracking (e.g., electromagnetic (EM) tracking, optical shape sensing (OSS), etc.).

Under such settings, a detected needle can be mapped into a same space with pre-identified targets. Thus, the spatial relationship between the needle and the target can be computed, based on which specific visual or auditory feedback is provided to the users both before and after needle firing. The present system and method provide intra-procedural-accuracy feedback to users immediately before and after the biopsy gun firing. With the feedback, a user can either adjust the needle to be in a better position for firing or take another sample if the target has already been missed. The feedback can be visual, auditory, or other feedback signals may be employed.

It should be understood that the present disclosure will be described in terms of medical instruments; however, the teachings are much broader and are applicable to any image based technology that involve alignment or activities. In somearrangements, the present principles are employed in tracking or analyzing complex biological or mechanical systems. In particular, the present principles are applicable to internal tracking procedures of biological systems and procedures in all areas of the body such as the lungs, gastro-intestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the disclosure.

Similarly, it will be appreciated that any flow charts, flow diagrams and the like represent various processes which may be substantially represented in computer readable storage media and so executed by a computer or processor, whether or not such computer or processor is explicitly shown.

Furthermore, arrangements can take the form of a computer

program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray^{™} and DVD.

Reference in the specification to "one embodiment" or "an embodiment" of the present principles, as well as other variations thereof, means that a particular feature, structure, characteristic, and so forth described in connection with the embodiment is included in at least one embodiment of the present principles. Thus, the appearances of the phrase "in one embodiment" or "in an embodiment", as well any other variations, appearing in various places throughout the specification are not necessarily all referring to the same embodiment.

It is to be appreciated that the use of any of the following "/", "and/or", and "at least one of", for example, in the cases of "A/B", "A and/or B" and "at least one of A and B", is intended to encompass the selection of the first listed option (A) only, or the selection of the second listed option (B) only, or the selection of both options (A and B). As a further example, in the cases of "A, B, and/or C" and "at least one of A, B, and C", such phrasing is intended to encompass the selection of the first listed option (A) only, or the selection of the second listed option (B) only, or the selection of the third listed option (C) only, or the selection of the first and the second listed options (A and B) only, or the selection of the first and third listed options (A and C) only, or the selection of the second and third listed options (B and C) only, or the selection of all three options (A and B and C). This may be extended, as readily apparent by one of ordinary skill in this and related arts, for as many items listed.

It will also be understood that when an element such as a layer, image, region or material is referred to as being "on" or "over" another element, it can be directly on the other element or intervening elements may also be present. In contrast, when an element is referred to as being "directly on" or "directly over" another element, there are no intervening elements present. It will also be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

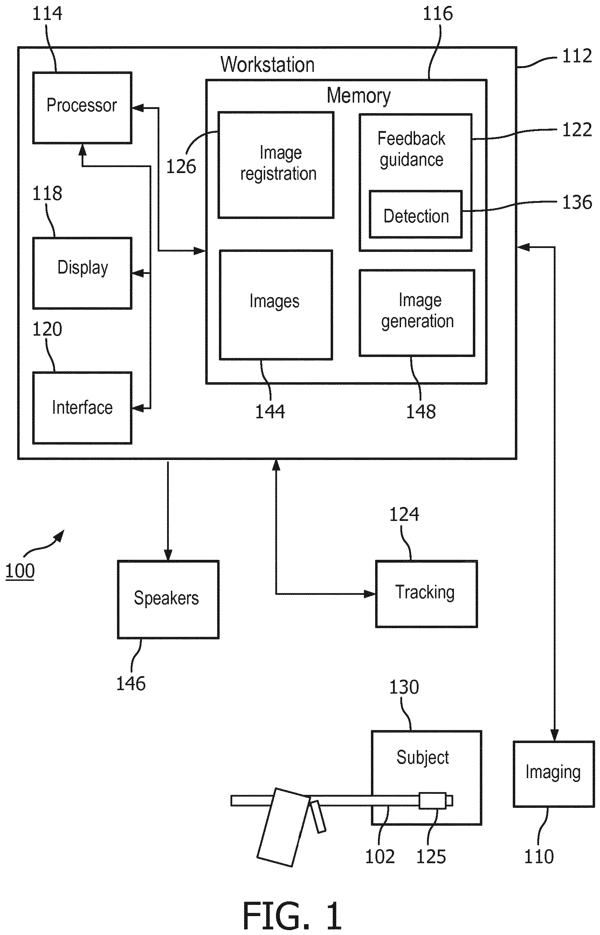

Referring now to the drawings in which like numerals represent the same or similar elements and initially to FIG. 1, a system 100 for instrument guidance using feedback before and after a trigger event is illustratively shown in accordance with an arrangement. System 100 may include a workstation or console 112 from which a procedure is supervised and/or managed. Workstation 112 preferably includes one or more processors 114 and memory 116 for storing programs and applications. The workstation 112 may include an imaging system 110 integrated therein or have the imaging system 110 independent from the workstation 112. The workstation 112 may be configured to provide other functions instead of or in addition to those described herein.

Memory 116 may store a feedback guidance application or module 122 configured to identify and track objects in an image. The feedback guidance application 122 can be run to detect an instrument 102 in an image, e.g., to assist in needle insertion and needle firing using live imaging. The feedback guidance application 122 includes a detection module or algorithm 136 configured to determine a position and orientation of the instrument 102 within an image. The live imaging may be collected using the imaging device or system 110. The imaging system 110 may include an ultrasound system, although other imaging modalities may be employed, e.g., fluoroscopy, etc.

The feedback guidance application 122 provides for live imaging only guided procedures and well as procedures with fused images (e.g., live images with stored static/preoperative images). The feedback guidance application 122 provides visual or audible signal warnings if the instrument 102 is deviating from a guideline (path determined for a biopsy needle or the like) or other stored criteria. In one example, the instrument 102 may include a biopsy gun. Before firing the biopsy gun, a display 118 can display a projected location of a biopsy core-taking portion based on a current position and orientation of a biopsy needle attached to the gun. The feedback guidance application 122 generates an actual tissue sample-taking area graphic before and/or after needle firing that can be displayed in an image shown on the display 118. Such graphics are employed as feedback for the proper alignment or positioning of the instrument 102 and/or the realignment or repositioning for a next task.

For image fusion guided procedures, a device tracking system 124 (with a sensor 125 (e.g., EM sensor, optical shape sensor, etc.) and an image registration module 126 may be employed to spatially map the detected instrument 102 (e.g., a needle) in 3D space (images 144). The feedback guidance application 122 computes the distance between a biopsy core location and a target according to the detected instrument 102. The feedback guidance application 122 provides signals for visual or audible feedback to users based on the distances between the biopsy core and the target. In the example of a biopsy, before firing, feedback on whether the target is on the needle insertion path and whether the needle is inserted to the correct depth to sample the target is provided. After firing, feedback on whether the biopsy core was actually taken from the targeted area is provided.

The instrument 102 may include a fired biopsy needle, other needles, a catheter, a guidewire, a probe, an endoscope, a robot, an electrode, a balloon device or other medical component, etc.

In an arrangement, an image generation module 148 is configured to generate objects to assist in the planning of a biopsy. The image generation module 148 generates overlays on displayed images to provide visual feedback to the user. In an arrangement, a biopsy guideline is generated by the image generation module 148 and projected in a real-time image, which is displayed on display 118.

The feedback guidance application 122 may use the geometric dimensions of the biopsy needle (specifically the distance between the needle tip and the biopsy-core taking part of the needle), and the estimate position and trajectory of a needle to determine a location of a core taken after a biopsy needle has fired. These features may be generated by the image generation module 148 and projected in the real-time image as feedback for the user. In another arrangement, acoustic information may be generated instead of or in addition to the visual feedback. A speaker or speakers 146 may be provided that receive audio signals from the feedback guidance application 122 to provide different forms of audio feedback. For example, the amplitude of an audio signal or its tone may be employed to indicate that a throw region is being approached or that the throw region has been exceeded. In another arrangement, textual information (on display 118) or audio commands (on speakers 146) may provide the same function by informing the user of the position based on measurements, etc. performed by the feedback guidance application 122.

Workstation 112 includes the display 118 for viewing internal images of a subject (patient) or volume 130 and may include images as an overlay or other rendering as generated by the image generation module 148. Display 118 may also permit a user to interact with the workstation 112 and its components and functions, or any other element within the system 100. This is further facilitated by an interface 120 which may include a keyboard, mouse, a joystick, a haptic device, or any other peripheral or control to permit user feedback from and interaction with the workstation 112.

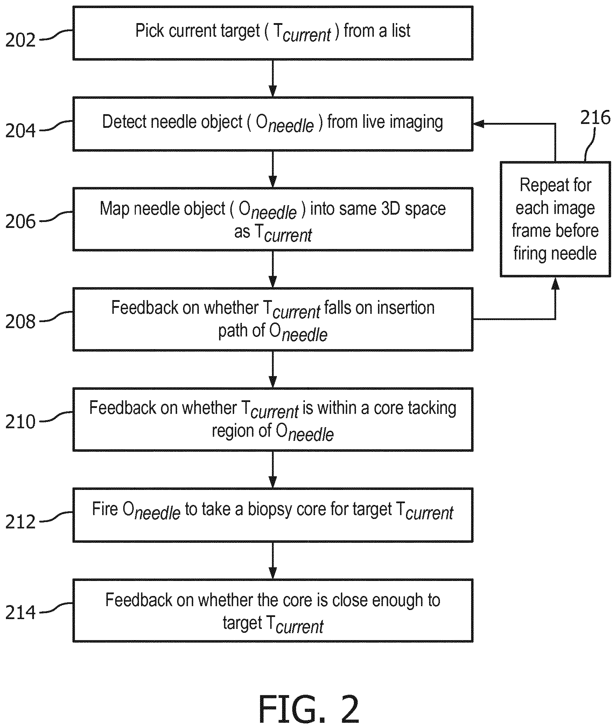

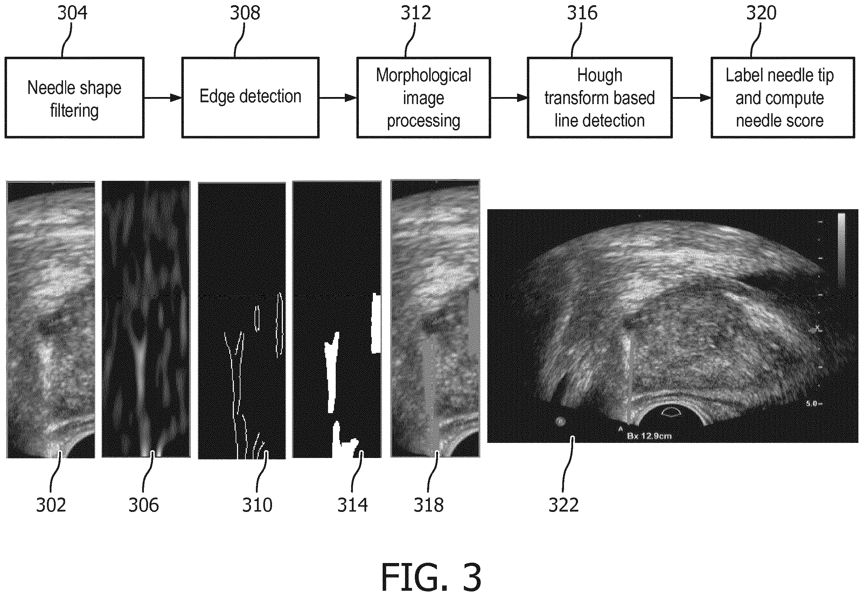

Referring to FIG. 2, a block/flow diagram is illustratively shown for a system/method using feedback in accordance with the feedback guidance application 122. An exemplary arrangement will be described using a needle for a biopsy procedure. It should be understood that other instruments may be employed for other procedures as well. For image fusion based targeted biopsy, a user picks a target (*T_{current}*) from a list of targets for biopsy in block 202. In block 204, once a biopsy needle enters a viewing field of a live imaging viewing pane or image, the needle object (*O_{needle}*) can be detected by using an image based detection method. Other methods may be employed as well, e.g., EM tracking, optical shapes sensing, etc. The detection can be triggered either automatically by continuously or intermittently monitoring imaging or manually by a user. An example of detecting a needle from an ultrasound image is described with reference to FIG. 3.

Referring to FIG. 3, a detection process (e.g., for detection module 136) may include the following system/method. An image 302 (e.g., ultrasound) containing a needle is given to a needle detection algorithm. The image 302 is filtered by a needle shape filter 304, which enhances tubular structures (like a needle) and suppresses other structures to provide a filtered image 306. An edge detection module 308 may perform edge detection for the filtered image 306 to extract the main enhanced areas in an edge detected image 310. Morphological image processing operations are then applied in block 312 to a binary edge image 314 for further processing. In block 316, a Hough transform is then employed to extract all the line segments from a processed binary image 318. In block 320, a line with highest possibility (highest needle score) to be the needle is picked as a final detection result. The needle tip is labeled in an image 322. The detection process described here may be employed as well as other image processing techniques, and the process can be generalized for needle detection with other imaging modalities (e.g., fluoroscopy, computed tomography, magnetic resonance, etc.).

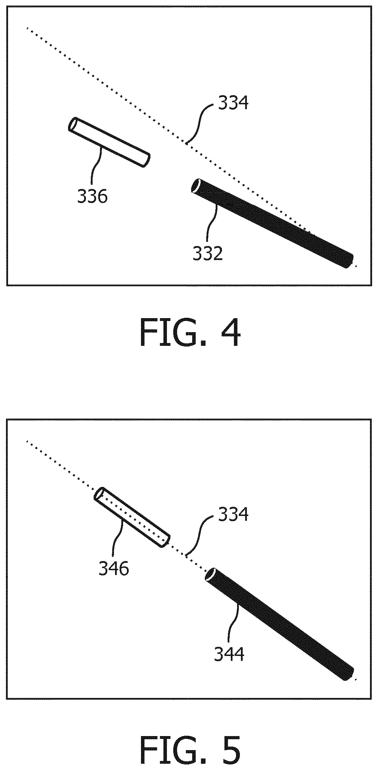

Referring again to FIG. 2, in block 206, for fusion guided targeted procedures, to correctly compute a distance to a target, the target and the detected needle need to be mapped into a common space (e.g., *T_{current}* 3D space). This can be achieved by using device tracking and image registration techniques. For example, electromagnetic (EM) tracking may be employed for tracking the ultrasound probe. By registering a reconstructed 3D ultrasound volume with a 3D MR volume, 2D ultrasound images can be mapped to the 3D MR space in real-time. Since the targets are identified from MR images, the transformation chain will bring the needle detected from 2D ultrasound images into the same MR imaging space as the targets. In addition, depending on how a biopsy guide is attached to an imaging device, biopsy guide bending or shifting may occur. In the presence of such an event, the needle will deviate from a biopsy guide line shown by the imaging equipment. This is illustratively depicted in FIG. 4.

In block 208, the system (100) checks whether the target falls on the pointing direction of the detected needle. Feedback is provided to the user, e.g., visual or audio feedback can be provided. For example, the biopsy guide line can be turned into a highlighted color when the current target falls on the line, or a sound can be played to confirm that user is pointing the needle in the right direction.

In block 210, once the needle is in the right direction, the system will continue to check whether the core taking part of the needle will cover the biopsy target once being fired. The 3D position of the core taking part, which is usually in the shape of a cylinder, is computed based on the anatomy of the needle, the needle tip location, and also the needle pointing direction. This is illustratively depicted in FIG. 5. For visual feedback, a marker can be put at the desired location for the needle tip along the needle. The user needs to insert the needle to that marked point for firing. Alternatively, a beeping sound can be played when the needle is getting close to the firing point. The frequency of the beeping may be used for denoting the distance between the needle tip and its desired location. In block 216, if feedback is received that the core taking is off target, the process is repeated by returning to block 204.

In block 212, when the user inserts the needle to the desired location, the needle can be fired to acquire a tissue sample. In block 214, the needle firing can be automatically detected or manually indicated. Automatic detection can be achieved by looking for the sudden increase of the needle length, since the firing is very fast, which can be captured by 1 to 3 frames of the live imaging depending on the frame rate of the system. With the firing detected, the distance between the actual biopsy core and the target can be computed in the same way as described above. If the system detects that the biopsy core is actually away from target but not covering it, a warning signal may be displayed on a screen or a warning sound may be played. Then, the user will have a chance to check the biopsy and redo it if the user determines that is necessary.

Referring to FIG. 4, an illustration is shown for providing feedback when a path of a needle 332 deviates from a projected biopsy guideline 334. The methods in accordance with the present principles can detect whether the actual needle is deviating from the projected biopsy guideline 334. Based on that information, either visual or audible feedback will be provided. A projected core taking region 336 is shown and is coupled with an orientation of the needle 332 at a distance from a tip of the needle.

Referring to FIG. 5, an estimated final location of a needle 344 for a firing based on the detected needle location can be superimposed over live or static images. With this feedback, users can know accurately where the needle 344 will end up after firing. This provides more accurate guidance than just virtual estimation based on a users' own knowledge and experience. An estimated or projected core taking region 346 is shown on the projected biopsy guideline 334 at an appropriate distance to account for firing of the needle 344.

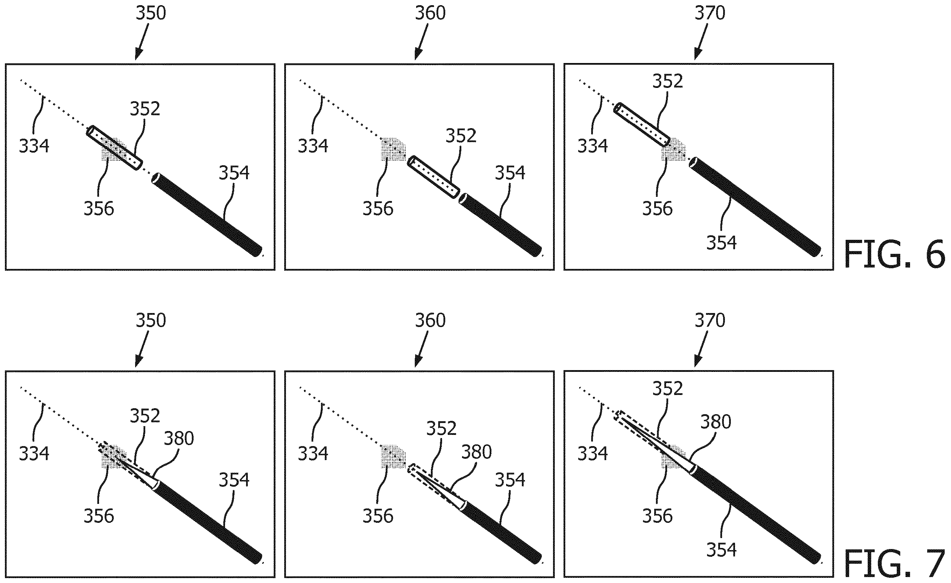

Referring to FIG. 6, an example of visual feedback on biopsy needle insertion depth is shown in accordance with the present principles. A needle 354 is detected and visual feedback is provided as to a distance from the needle where a core sample will be taken. In instance one 350, a core projection 352 coincides well with a target 356 to be biopsied. In instance two 360, the core projection 352 is too shallow. In instance three 370, the core projection 352 is too deep. The present systems and methods provide feedback to users before and after the biopsy gun firing by determining the spatial relationship between the biopsy needle and the target. This enables users to take appropriate actions to improve the accuracy and to achieve a higher success rate.

Referring to FIG. 7, an example of visual feedback on biopsy needle insertion depth is shown in accordance with another embodiment. The needle 354 includes a representation of a core-taking portion 380 as visual feedback for instances 350, 360 and 370.

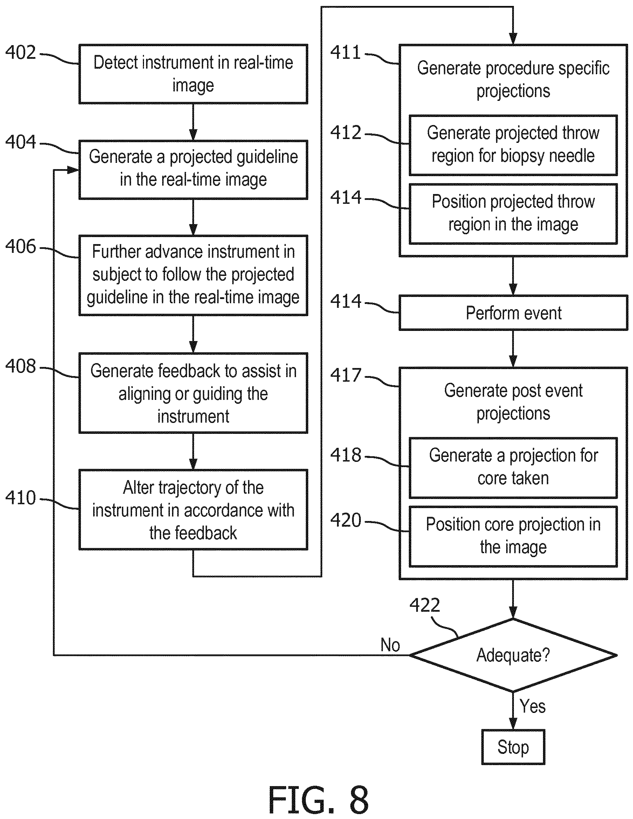

Referring to FIG. 8, a method for instrument guidance is illustratively shown. In block 402, an instrument is inserted and detected in a real-time image. The real-time image may include an ultrasound image, although other real-time image may be employed, e.g., fluoroscopic images, etc. The instrument may be detected using a detection algorithm to determine instrument position in the image.

In block 404, a projected guideline is generated in the image to indicate a path for the instrument in a subject. In block 406, the instrument is advanced further or aimed in the subject, if needed, to attempt to follow the guideline. The instrument may be partially or completely inserted in block 402. If partially inserted, the instrument may be further advanced here.

In block 408, feedback for aligning or guiding the instrument is generated in accordance with a detected event. The feedback may include audio and/or visual feedback to provide guidance to a user in positioning the instrument in the area of interest relative to the projected guideline in real-time. The audio and visual feedback may include, e.g., an alert on a display, a sound, a change in frequency of a sound, a change in the projected guideline, etc. The detected event may include misalignment of the instrument from a projected path, proximity to a target, a display of a projected core taking region in the area of interest and/or a display of a region of an actual core taken from the area of interest after firing a biopsy needle.

In block 410, a trajectory of the instrument is altered in accordance with the feedback. The feedback and altering the trajectory continues until alignment is achieved.

In block 411, procedure specific projections are generated in the image. For example, the instrument may include a biopsy needle having a throw for a core taking portion. In block 412, a projected region is generated representing the throw in the displayed image as visual feedback). In block 414, the projected region is positioned in the image.

In block 416, an instrument event is performed. For example, the needle is fired to collect a sample.

In block 417, post event projections are generated. In block 418, a core projection region is generated representing a core taken from the subject. In block 420, the core projection region is positioned on the target in the image.

In block 422, a decision as to the adequacy of the procedure (e.g., core taken) is determined. If adequate, stop; otherwise return to block 404.

As described, before firing the needle, the (un-fired) needle tip is detected and the throw is added to estimate the location of the biopsy core if the needle were fired at that moment. Feedback is provided on whether the needle is positioned correctly to sample the target. After firing, the (fired) needle tip is detected and a dead zone (between core-taking portion and needle tip) is subtracted to estimate where the tissue was actually sampled. Feedback is provided on whether the target was correctly sampled, or whether it may need re-sampling.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described arrangements for intra-procedural accuracy feedback for image-guided biopsy (which are intended to be illustrative and not limiting), it is noted that modifications and variations can be made by persons skilled in the art in light of the above teachings. It is therefore to be understood that changes may be made in the particular arrangements of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. A feedback system for biopsy gun guidance the biopsy gun comprising a fireable biopsy needle having a needle tip, the biopsy needle, when fired, shoots forward to obtain a tissue sample from a location that is offset from the needle tip by a fixed distance, the system comprising:
a feedback guidance module (122) configured to detect the biopsy needle in a real-time image and generate feedback including proximity to a target for aligning or guiding the instrument; and
an image generation module (148) configured to generate a projected guideline in the image and a projected location of a biopsy core-taking portion in the form of a graphic of an actual tissue sample-taking area based on a detected current position and orientation of the biopsy needle and the fixed distance offset from the needle tip;
the feedback guidance module configured to generate at least one of audio and visual feedback based on distances between the detected biopsy needle and
(i) the projected guideline;
(ii) the graphic of an actual tissue sample-taking area and the target;
to provide guidance to a user in positioning the biopsy needle relative to the target in real-time.

2. The system as recited in claim 1, wherein the at least one of audio and visual feedback includes an alert on a display, a sound, a change in frequency of a sound and/or a change in the projected guideline.

3. The system as recited in claim 1, wherein the at least one of audio and visual feedback includes a display of a region of an actual core taken (380) from an area of interest after firing the biopsy needle.

4. The system as recited in claim 1, wherein the feedback guidance module includes an instrument detection algorithm (136) to determine a position of the biopsy needle in the image.

5. The system according to claim 1, comprising:
an imaging system (110) configured to capture real-time images for a region of interest, the region of interest including the biopsy needle (102) having a core taking portion extendable from the needle; and
a workstation (112) including a processor (114) and memory (116);
wherein the feedback guidance module (122) and image generation module are stored in the workstation memory.

6. The system as recited in claim 5, wherein the guidance provided to the user comprises at least one of: misalignment of the needle from a projected path, proximity to the target, display of the projected core taking region in the area of interest and/or a display of a region of an actual core taken from the region of interest after firing the biopsy needle.

7. The system as recited in claim 5, wherein the feedback guidance module includes a detection algorithm (136) to determine a position of the biopsy needle in the image.

## Patentansprüche

1. Rückmeldungssystem zur Führung einer Biopsiepistole, wobei die Biopsiepistole eine abfeuerbare Biopsienadel umfasst, die eine Nadelspitze aufweist, wobei die Biopsienadel, wenn sie abgefeuert wird, nach vorne schießt, um eine Gewebeprobe von einer Stelle zu erhalten, die um einen festen Abstand von der Nadelspitze versetzt ist, wobei das System umfasst:
ein Rückmelde-Führungsmodul (122), das konfiguriert ist, um die Biopsienadel in einem Echtzeitbild zu erkennen und eine Rückmeldung zu erzeugen, die Nähe zu einem Ziel zum Ausrichten oder Führen des Instruments beinhaltet; und
ein Bilderzeugungsmodul (148), das konfiguriert ist, um eine projizierte Führungslinie im Bild und eine projizierte Stelle eines Abschnitts zur Entnahme eines Biopsie-Kerns in Form einer Grafik eines tatsächlichen Bereichs zur Entnahme einer Gewebeprobe basierend auf einer erkannten aktuellen Position und Ausrichtung der Biopsienadel und dem Versatz um einen festen Abstand von der Nadelspitze zu erzeugen;
wobei das Rückmelde-Führungsmodul konfiguriert ist, um mindestens eine von akustischer und visueller Rückmeldung basierend auf Abständen zwischen der erkannten Biopsienadel und Folgendem zu erzeugen
(i) der projizierten Führungslinie;
(ii) der Grafik eines tatsächlichen Bereichs zur Entnahme einer Gewebeprobe und des Ziels;
um einem Benutzer beim Positionieren der Biopsienadel in Bezug auf das Ziel in Echtzeit Führung bereitzustellen.

2. System nach Anspruch 1, wobei die mindestens eine von akustischer und visueller Rückmeldung eine Warnung auf einer Anzeige, einen Ton, eine Änderung der Frequenz eines Tons und/oder eine Änderung der projizierten Führungslinie beinhaltet.

3. System nach Anspruch 1, wobei die mindestens eine von akustischer und visueller Rückmeldung eine Anzeige eines Bereichs eines tatsächlich entnommenen Kerns (380) aus einem Bereich von Interesse nach Abfeuern der Biopsienadel beinhaltet.

4. System nach Anspruch 1, wobei das Rückmelde-Führungsmodul einen Instrumentenerkennungsalgorithmus (136) beinhaltet, um eine Position der Biopsienadel im Bild zu bestimmen.

5. System nach Anspruch 1, umfassend:
ein Bildgebungssystem (110), das konfiguriert ist, um Echtzeitbilder eines Bereichs von Interesse aufzunehmen, wobei der Bereich von Interesse die Biopsienadel (102) beinhaltet, die einen aus der Nadel ausfahrbaren Abschnitt zur Kernentnahme aufweist; und
eine Arbeitsstation (112), die einen Prozessor (114) und einen Speicher (116) beinhaltet;
wobei das Rückmelde-Führungsmodul (122) und das Bilderzeugungsmodul im Speicher der Arbeitsstation gespeichert sind.

6. System nach Anspruch 5, wobei die dem Benutzer bereitgestellte Führung mindestens eines umfasst von: Fehlausrichtung der Nadel von einem projizierten Pfad, Nähe zum Ziel, Anzeige des projizierten Kernentnahmebereichs im Bereich von Interesse und/oder eine Anzeige eines Bereichs eines tatsächlichen Kerns, der nach Abfeuern der Biopsienadel aus dem Bereich von Interesse entnommen wurde.

7. System nach Anspruch 5, wobei das Rückmelde-Führungsmodul einen Erkennungsalgorithmus (136) beinhaltet, um eine Position der Biopsienadel im Bild zu bestimmen.

## Revendications

1. Système de rétroaction pour un guidage de pistolet de biopsie, le pistolet de biopsie comprenant une aiguille de biopsie pouvant être tirée qui présente une pointe d'aiguille, l'aiguille de biopsie, lorsqu'elle est tirée, se projetant vers l'avant pour obtenir un échantillon de tissu à partir d'un emplacement qui est décalé d'une distance fixe par rapport à la pointe de l'aiguille, le système comprenant :
un module de guidage à rétroaction (122) configuré pour détecter l'aiguille de biopsie dans une image en temps réel et générer une rétroaction incluant la proximité d'une cible pour aligner ou pour guider l'instrument ; et
un module de génération d'image (148) configuré pour générer une ligne de guidage projetée dans l'image et un emplacement projeté d'une partie de carottage biopsique sous la forme d'un graphique d'une zone effective de prélèvement d'échantillon de tissu sur la base d'une position et d'une orientation actuelles détectées de l'aiguille de biopsie et du décalage d'une distance fixe par rapport à la pointe de l'aiguille ;
le module de guidage à rétroaction configuré pour générer au moins une rétroaction auditive et/ou visuelle sur la base de distances entre l'aiguille de biopsie détectée et
(i) la ligne de guidage projetée ;
(ii) le graphique d'une zone effective de prélèvement d'échantillon de tissu et de la cible ;
pour fournir un guidage à un utilisateur dans le positionnement de l'aiguille de biopsie par rapport à la cible en temps réel.

2. Système selon la revendication 1, dans lequel la au moins une rétroaction auditive et/ou visuelle inclut une alerte sur un écran d'affichage, un son, un changement de fréquence d'un son et/ou un changement de la ligne de guidage projetée.

3. Système selon la revendication 1, dans lequel la au moins une rétroaction auditive et/ou visuelle inclut un affichage d'une région d'une carotte effective prélevée (380) à partir d'une zone d'intérêt après le tir de l'aiguille de biopsie.

4. Système selon la revendication 1, dans lequel le module de guidage à rétroaction inclut un algorithme de détection d'instrument (136) pour déterminer une position de l'aiguille de biopsie dans l'image.

5. Système selon la revendication 1, comprenant :
un système d'imagerie (110) configuré pour capturer des images en temps réel pour une région d'intérêt, la région d'intérêt incluant l'aiguille de biopsie (102) présentant une partie de carottage extensible à partir de l'aiguille ; et
un poste de travail (112) incluant un processeur (114) et une mémoire (116) ;
dans lequel le module de guidage à rétroaction (122) et le module de génération d'image sont stockés dans la mémoire du poste de travail.

6. Système selon la revendication 5, dans lequel le guidage fourni à l'utilisateur comprend au moins l'un : d'un mauvais alignement de l'aiguille par rapport à une trajectoire projetée, d'une proximité de la cible, d'un affichage de la région de carottage projetée dans la zone d'intérêt et/ou d'un affichage d'une région d'une carotte effective prélevée dans la région d'intérêt après le tir de l'aiguille de biopsie.

7. Système selon la revendication 5, dans lequel le module de guidage à rétroaction inclut un algorithme de détection (136) pour déterminer une position de l'aiguille de biopsie dans l'image.
